# EUROPÄISCHE PATENTSCHRIFT

(11) **EP 1 147 419 B1**
(45) Veröffentlichungstag und Bekanntmachung des Hinweises auf die Patenterteilung: **29.10.2003**
(21) Anmeldenummer: 99952414.3
(22) Anmeldetag: 12.08.1999
(51) Int. Cl.: G01N 33/569, G01N 33/566, G01N 33/58, G01N 33/543

(54) **NACHWEIS VON ZELLEN MITTELS SPEZIFISCHER ZELLWAND-BINDENDER DOMÄNEN (CBD) VON ZELLWAND-BINDENDEN PROTEINEN**
LABELING, IMMOBILISATION, ENRICHMENT, PURIFICATION AND DETECTION OF CELLS USING SPECIFIC DOMAINS THAT BIND TO THE CELL WALLS (CBD) OF PROTEINS BINDING TO THE DRAWN CELL WALLS OF VIRUSES, BACTERIA OR EUKARYOTIC CELLS
MARQUAGE, IMMOBILISATION, ENRICHISSEMENT, PURIFICATION ET DETECTION DE CELLULES A L'AIDE DE L'UTILISATION DE DOMAINES SPECIFIQUES SE LIANT A DES PAROIS CELLULAIRES (CBD) DE PROTEINES SE LIANT A DES PAROIS CELLULAIRES TIREES DE VIRUS, BACTERIES OU CELLULES EUCARYOTES

(30) Priorität: 20.08.1998 DE 19837751
(43) Veröffentlichungstag der Anmeldung: 24.10.2001
(73) Patentinhaber: Scherer, Siegfried, Prof. Dr., 85354 Freising (DE); Loessner, Martin, Dr., 85417 Marling (DE)
(72) Erfinder: Scherer, Siegfried, Prof. Dr., 85354 Freising (DE); Loessner, Martin, Dr., 85417 Marling (DE)
(74) Vertreter: HOFFMANN - EITLE
(86) Internationale Anmeldenummer: DE9902562
(87) Internationale Veröffentlichungsnummer: WO00011472

(56) Entgegenhaltungen:
- WO-A-88/02781
- WO-A-96/07756
- DE-C- 4 326 617
- US-A- 4 329 151

## Beschreibung

Die Erfindung betrifft ein Verfahren und eine Reagenzienzusammenstellung zur Spezifischen Erkennung von Zielzellen durch Bindung mittels der Verwendung spezifischer Zellwand-bindender Domänen (CBD) von Zellwand-bindenden Proteinen aus Viren, Bakterien oder eukaryontischen Zellen, und die Verwendung des Verfahrens zur Markierung, Immobilisierung, Anreicherung, Reinigung und Nachweis von Zellen gemäß den unabhängigen Ansprüchen 1, 22 und 23. Bevorzügte Ausführungsbeispiele sind in den Unteransprüchen definiert.

### Stand der Technik

Für den Nachweis und die Bestimmung von Zellen und Dauerformen von Zellen (Sporen) von Mikroorganismen (Bakterien, Hefen, Schimmel) und eukaryontischen Zellen (nachfolgend zusammenfassend bezeichnet als Zellen, beziehungsweise Zielzellen) werden als nachzuweisende Zielmoleküle neben von den Zellen ausgeschiedenen Metaboliten und/oder anderen zellulären Bestandteilen oder Molekülen häufig die Zellen selber verwendet. Hierzu werden meist spezielle Antikörper (Proteine aus der Klasse der Immunglobuline) verwendet. Antikörper "erkennen" spezielle Antigene auf der Oberfläche der Zielorganismen und können reversibel daran binden. Diese Bindung kann durch eines oder mehrere dem Fachmann bekannte Verfahren sichtbar gemacht werden, z.B. durch direkte Verbindung des Antikörpers mit (fluoreszierenden) Farbstoffen (z.B. Fluoreszein), biologischen Markermolekülen, partikulären Markem (z.B. Goldpartikel), oder verschiedensten Amplifikatoren (hochaffine Bindemoleküle (z.B. Biotin, wird gebunden von Avidin), oder Enzymen (z.B. Peroxidase, Phosphatase)), welche dann wiederum eine Reaktion katalysieren, welche entweder ein sichtbares Farbstoffpräzipitat, eine sichtbare Farbstoffabspaltung, oder eine sichtbare Licht-emittierende Reaktion (Chemilumineszenz, Biolumineszenz) bereitstellt Eine weitere Möglichkeit ist die Verwendung eines sekundären (markierten) Antikörpers welcher den (in diesem Falle nicht markierten, primären) Antikörper erkennt (z.B. ein Anti-Kaninchen Antikörper aus der Ziege), daran bindet, und selber mittels Konjugation mit oben erwähnten Goldpartikeln, Farbstoffen, oder eine messbare Reaktion katalysierende Enzyme die messbare (Nachweis)-Reaktion ausführt. Auch möglich ist hier die Verwendung von sekundären Bindemolekülen welche nicht zur Klasse der Immunglobuline gehören, wie z.B. Avidin (kann an Biotin-markierten primären Antikörper binden), oder Protein A aus *Staphylococcus aureus*, welches generell an bestimmte Klassen der Immunglobuline bindet Diese Bindemoleküle können wiederum mit einem oder mehreren einer Vielzahl von Nachweismolekülen (Amplifikatoren, siehe oben) verbunden sein.

Eine weitere Möglichkeit ist die Immobilisierung der nachzuweisenden Zielmoleküle oder Zellen an einer festen Phase (z.B. Oberflächen-behandelte Kunststoff Mikrotiterplatten, oder entsprechend behandelte sogenannte Dip-Sticks). Hier werden mittels spezifischer Antikörper, welche an die feste Phase gebunden sind, die Zielzellen selektiv aus einem heterogenen Gemisch heraus gebunden. Nach einem Waschschritt zur Entfernung der ungebundenen Zielmoleküle (Antigene, Zellen) wird nachfolgend ein zweiter, diesmal freier und mit oben erwähnten Farbstoffen, Markermolekülen, partikulären Markern, radioaktiven Isotopen, oder Amplifikatoren (z.B. Enzyme, Biotin) verbundener Antikörper verwendet, um ebensfalls an die nun auch an die feste Phase immobilisierten Antigene (Moleküle oder Zielzellen) zu binden, und diese auf die oben beschriebene Art nachzuweisen. Diese Art und Durchführung der Nachweisreaktion wird als Sandwich-ELISA (Enzyme-linked-Immunosorbent assay) bezeichnet.

Eine weitere Anwendung von spezifischen Antikörpern im Rahmen der Immobilisierung ist die Anreicherung von Zielzellen durch eine bewegliche feste Phase. So lassen sich z.B. Antikörper oder Lektine an kleine ferromagnetische Partikel koppeln, welche nach Bindung des Zielantigens in flüssiger Phase mit Hilfe eines magnetischen Feldes aus dieser isoliert werden können, und somit eine selektive Anreicherung von Zielmolekülen oder Zielzellen mit weiteren, nachfolgenden Bearbeitungsschritten erlauben.

Diese oben beschriebenen Verfahren auf Antikörper-Basis haben einige Nachteile: Es müssen spezifische Antikörper hergestellt werden, zunächst durch Immunisierung von Tieren mit dem Zielantigen oder einem Bestandteil desselben. Es ist hier nicht sichergestellt das die gewünschte Immunantwort auch erzielt wird. Falls das Serum nach einiger Zeit einen genügend hohen Titer an spezifischen Antikörpem gegen das Zielantigen enthalten sollte, können diese aus dem Serum der Tiere gewonnen werden, mittels dem Fachmann bekannter Methoden zur Aufreinigung von Immunglobulinen. Diese Antikörper, unabhängig von ihrem Reinheitsgrad, werden als polyklonale Antikörper bezeichnet. Unerwünschte Kreuzreaktionen mit fremden, den Zielantigen nicht unbedingt verwandten Antigenen werden häufig beobachtet und stellen einen wesentlichen Nachteil dar. Desweiteren ist es möglich, durch dem Fachmann bekannte Fusionstechniken von einzelnen Antikörper-produzierenden Immun-B-Zellen und einer Tumor-Zellinie zu Hybridom-Zellen sogenannte monoklonale Antikörper in Zellkultur zu gewinnen. Diese Hybridomatechniken sind jedoch langwierig und aufwendig. Nachfolgend müssen gegebenenfalls in weiteren Reaktionen an die gereinigten Antikörper noch Farbstoffe, partikuläre Marker, oder Amplifikatoren gekoppelt werden, und die Reaktionsprodukte gereinigt werden. Zudem ist es nicht in allen Fällen ohne weiteres möglich einen speziellen Antikörper gegen das Zielantigen zu gewinnen, besonders wenn es sich hierbei um ganze, intakte Zellen handelt. Ein weiterer wesentlicher Nachteil der benutzng von Antikörpem ist die relativ lange Reaktionszeit welche zur Bindung des Antikörpers and das Zielantigen benötigt wird. Dieses kann bis zu 12 Stunden dauern.

Derartige, oben beschriebene Verfahren, sowie Verfahrensvarianten sind dem Fachmann geläufig und in der Literatur beschrieben. Erfindungsgemäß werden diese Reaktionen als übliche Nachweisverfahren zusammengefaßt Die Reagenzien, die für diese Nachweisverfahren notwendig sind, werden als übliche Nachweismittel bezeichnet

Proteine oder Enzyme welche selektiv Zellwände von Zellen spalten sind aus der Literatur in unterschiedlicher Form bekannt. Es ist bekannt das solche Proteine in den meisten Fällen eine Domänen-Struktur aufweisen, wobei ein Teilabschnitt der Polypeptidkette in der nativen, fertig gefalteten Tertiärstruktur in der Lage ist, bestimmte Moleküle oder Molekül-Konformationen auf der Oberfläche von Zellen zu erkennen und an diese zu binden. Solche Moleküle sind die Bakteriophagen-kodierten Zellwandhydrolasen (Microbiol. Rev. 56, Seite 430-481 (1992)); bakterielle Zellwandhydrolasen wie z.B. Lysostaphin (Mol. Gen. Genet. 209, Seite 563-569 (1987)), und verschiedene Autolysine. Außerdem können an Zellwände binden die Rezeptor-Moleküle kodiert von der DNS von Bakteriophagen und anderen Viren spezifisch für Hefen, Schimmel und eukaryontische Zellen; und auch alle mit der Zellwand von Zellen nicht-kovalent assoziierten Zellwandproteine kodiert von der zelleigenen DNS.

Aufgabe der hier offengelegten Erfindung ist es, Verfahren bereitzustellen, bei denen die Zielzellen mittels der Verwendung definierter Polypeptide (Protein-Domänen) von bekannter Aminosäure Sequenz spezifisch erkannt und durch Bindung der Polypeptide an diese Zellen spezifisch gebunden und markiert werden können. Diese definierten Polypeptide sind spezielle Unterabschnitte (Zellwand-bindende Domänen, engl.: Cell wall-binding domains, im weiteren als CBD bezeichnet) von zellwandständigen und zellwandbindenden Proteinen oder enzymatisch aktiven Proteinen (Enzymen) von Zellen oder Viren, sowie Kombinationen derselben miteinander. Die für diese CBD kodierenden Genabschnitte werden aus den entsprechenden für die Zellwand-bindenden Proteine kodierenden Erbinformationen der Zellen oder Viren ermittelt. Diese entsprechenden Teilabschnitte werden dann mittels dem Fachmann bekannten molekularbiologischen Techniken isoliert und vermehrt. Sodann werden diese Genabschnitte mittels dem Fachmann bekannten Techniken mit neuen Signalen (für Transkription und Translation) und Replikationsstrukturen (z.B. Plasmiden) kombiniert, welche die unabhängige Herstellung dieser Proteinfragmente (Polypeptide) in heterologen Organismen ermöglicht. Diese rekombinierten Genabschnitte, kodierend für (neuartige) Proteine, werden mittels dem Fachmann bekannten Techniken in geeignete Organismen eingebracht (z.B. *Escherichia coli* Bakterien oder *Pichia pastoris* Hefen), zur Gewinnung der rekombinanten Genprodukte (Proteine, Polypeptide). Anschließend oder auch während der rekombinanten Eipression sollen die Polypeptidketten gekoppelt werden mit geeigneten partikuläre Markern, enzymatischen Amplifikatoren, Farbstoffen, Isotopen oder Markergenen, wie z.B. fluoreszierenden Proteinen (z.B. Grün-fluoreszierendes-Protein (GFP) aus der Qualle *Aequoria victoria*; Science 263, Seite 802-805 (1994); oder vorzugsweise modifiziertes GFP mit erhöhter Emissionsintensität (GFP-mut1, GFP-mut2); Gene 173, Seite 33-38 (1996), oder auch Blau-fluoreszierendes Protein (BFP) oder anderen fluoreszierenden Proteinen.

CBD Polypeptide haben zu spezifischen, Zellwand-bindenden Antikörpern vergleichbare Eigenschaften. Sie erkennen spezifische Epitope (Proteine, Kohlenhydrate, Lipide, oder Verbindungen mit denselben) auf oder in der Zellwand und binden an diese Epitope. CBD Polypeptide haben jedoch Vorteile in der einfachen und kostengünstigen Herstellung. Ein wesentlicher Unterschied und Vorteil von CBD Polypeptiden gegenüber Antikörpern der Klasse der Immunglobuline ist die Möglichkeit der direkten Verbindung mit geeigneten Reportermolekülen mittels genetischer Fusion der kodierenden DNS Sequenz für CBD zu DNS Sequenzen kodierend für Reportermoleküle und/oder Amplifikatoren wie zum Beispiel GFP Proteine, oder enzymatisch aktiven Amplifikatoren. Dieses Verfahren ermöglicht die Bereitstellung von Fusionsproteinen aus CBD und erwähnten Reportermolekülen und oder Amplifikatoren, mit entsprechend unterschiedlich funktionalen Domänen für die Erkennung und Bindung an die Zielzellen (CBD Domäne), sowie die Sichtbarmachung und Möglichkeit zur Detektion dieser spezifischen Bindung durch die Reportermolekül/Amplifikator-Domäne.

Gegenstand der Erfindung sind Verfahren zur spezifischen Sichtbarmachung und zum spezifischen Nachweis von Zellen in einer Probe (diagnostische Verfahren) durch die spezifische Bindung von CBD an definierte Zielzellen und durch eine darauf folgende Nachweisreaktion, welche vermittelt wird von an die speziellen CBD gekoppelten geeigneten partikulären Marker, Amplifikatoren, Farbstoffe, Isotopen oder vorzugsweise fluoreszierende Proteine (vorzugsweise modifiziertes GFP). Diese Kopplung kann entweder durch direkte translationelle Fusion geschehen, oder durch Modifikation der CBD nach Herstellung und Reinigung der Polypeptide.

Gegenstand der Erfindung ist weiterhin die Verwendung von CBD Polypeptiden für Verfahren zur spezifischen Immobilisierung von Zellen, wobei die CBD Polypeptide mittels dem Fachmann bekannter Verfahren an eine feste Phase gebunden werden und nach Kontakt mit einer die Zielzellen enthaltenden (vorzugsweise flüssigen) Probe durch ihre spezifische Bindefähigkeit an die Zellwand der Zellen diese an die feste Phase immobilisieren. In einem nachfolgenden Schritt kann das Vorhandensein immobilisierter Zielzellen nun mittels markierter CBD (wie oben ausgeführt) nach Art eines Sandwich-CBD Assays nachgewiesen werden. Wenn als feste Phase magnetische Partikel verwendet werden, eignet sich das Immobilisierungs-Verfahren auch zur selektiven Anreicherung von Zellen aus einem heterogenen Gemisch von anderen Zellen und sonstigen Fremdstoffen.

Gegenstand der Erfindung sind schließlich Reagenz-Zusammenstellungen (Kits) geeignet für die oben bezeichneten spezifischen Verfahren zur Markierung, zum Nachweis, zur Immobilisierung und zur Anreicherung von Zellen, enthaltend neben den üblichen Nachweismitteln ein oder mehrere gereinigte oder an feste Phasen gebundene spezielle CBD Polypetide oder Verbindungen mit denselben.

Beispiele für dem Fachmann bekannte Bakteriophagen-kodierte Zellwandhydrolasen, bakterielle Zellwandhydrolasen, Autolysine, die Rezeptor-Moleküle kodiert von der DNS von Bakteriophagen und anderen Viren spezifisch für Hefen, Schimmel und eukaryontische Zellen, und auch alle mit der Zellwand von Zellen nicht-kovalent assoziierten Zellwandproteine kodiert von der zelleigenen DNS werden in den oben genannten Dokumenten und in der bekannten Fachliteratur offenbart. Zellwand-hydrolysierende Enzyme von Bakteriophagen (Phagenlysine, Endolysine, Lysine) können sehr spezifisch für die Hydrolyse der bakteriellen Zellwand sein. Zu diesen Enzymen gehören solche Lysine, die von Bakteriophagen für Bakterien der Gattung *Listeria* kodiert und während der späten Genexpression in der Phagenvermehrung gebildet werden. In DE 43 26 617 wird beispielsweise die Verwendung von Lysinen aus *Listeria* Phagen als spezifisches Mittel zur Bekämpfung von Listerien offenbart; in DE 195 06 615 wird die Verwendung von Phagenlysinen zum spezifischen Zellaufschluß beschrieben. Die Gene für diese Phagenlysine sind bekannt (Mol. Microbiol. 16, Seite 1231-1241 (1995); Microbiology 141, Seite 2577-2584 (1995).

In US 4,329,151 wird die Verwendung von nicht-abgebauten Streptolysin-O Molekülen, die auf Polystyrol-Latex-Partikel adsorbiert sind, zur Bestimmung von pathogenen Infektionen beschrieben.

Es ist möglich, die Zellwand-bindenden Domänen (CBD) innerhalb der Struktur beispielsweise dieser Phagenlysine zu bestimmen, über verschiedene Methoden, beispielsweise Homologievergleiche der kodierenden Gene (Nukleotidsequenzen), oder der Genprodukte (Aminosäuresequenzen), oder durch die unabhängige Expression von Teilabschnitten der kodierenden Gene in Kulturen von rekombinanten Bakterien, mit nachfolgender Bestimmung der Zellwand-Bindefähigkeit der vorliegenden, individuellen Fragmente des ursprünglichen Proteinmoleküls. Wichtig ist hierbei daß jegliche hydrolytische Aktivität der einzelnen gewonnenen Fragmente durch die alleinige Expression der CBD Domäne fehlt, oder auch gezielt durch Mutationen oder Deletionen zerstört wird.

### Beispiel

Das folgende Beispiel soll den Erfindungsgedanken näher erläutern; es stellt keine Einschränkung des Erfindungsumfanges dar.

### Markierung und Nachweis von Listeria Zellen.

Erfindungsgemäß konnte die CBD im Carboxy-Terminalen Bereich des Enzyms Ply500, kodiert vom *Listeria* Phagen A500, lokalisiert werden. Dazu wurden zuerst Vergleiche der Aminosäuresequenz von Ply500 mit allen anderen bekannten Proteinen in den gängigen, allgemein zugänglichen Datenbanken (GenBank, SwissProt) durchgeführt Es zeigte sich das der Amino-terminale Teil des Proteins Ähnlichkeiten zu den enzymatisch aktiven Domänen von anderen zellwandhydrolytischen Enzymen aufweist, während der Carboxy-terminale Teil völlig eigenständig zu sein scheint. Dann wurden mittels spezieller Oligonukleotid-Primer und der Polymerase-Kettenrektion definierte Fragmente des kodierenden Genes (*ply500*) gewonnen, welche am 5'-Ende mit eigener Ribosomen-Bindestelle und ATG Start Kodon versehen sind. Dieses Verfahren ermöglicht die unabhängige Expression dieser Genefragmente in heterologen Organismen wie dem Bakterium *Escherichia coli*. Die erhaltenen Genfragmente wurden in einen geeigneten Plasmid-Vektor (pSP72) eingeführt und in *E. coli* transformiert. Nach Induktion der Expression und testen der synthetisierten Genprodukte auf lytische Aktivität gegenüber *Listeria*-Zellen mit Hilfe eines Bioassays zeigte sich das nur das ganze, intakte Gen hydrolytisch aktiv ist Der Amino-terminale Teil von Ply500 konnte ohne den Carboxy-terminalen Teil *Listeria* Zellwände nicht spalten. Die Carboxy-terminale Domäne (Aminosäure His133 bis Lys289) sollte also die Bindung an die Zellwand ermöglichen, und stellt eine mögliche CBD dar. Nun wurde mit Hilfe von dem Fachmann bekannter molekularbiologischer Techniken und Klonierungverfahren eine genetische Fusion der kodierenden Nukleotidsequenzen für Grün-fluoreszierendes Protein (GFP) mit derjenigen für CBD von Ply500 hergestellt, in einen Expressionsvektor unter Kontrolle eines induzierbaren Promoters einkloniert, und in Bakterien der Gattung *E. coli* transformiert. Das aus der Expression des Fusionsgens in den Bakterien gebildete Genprodukt stellt ein Fusionsprotein aus einer Polypeptidkette dar, wobei das GFP den aminoterminalen Teil darstellt und die Ply500 CBD den carboxyterminalen Teil bildet. Das Protein kann nach der Synthese in rekombinanten Bakterien und deren Aufschluss durch geeignete Methoden mittels dem Fachmann bekannter Methoden aus den Zell-Lysaten gereinigt und für nachfolgende Versuche bereitgestellt werden. Das gereinigte Fusionsprotein GFP-CBD500 wird in einem wässrigen Puffer aufgenommen (z.B. 50 mM TrisCl, pH 8,0, 200 mM NaCl, 5 mM MgSO₄), und kann bei -20° C gelagert werden.

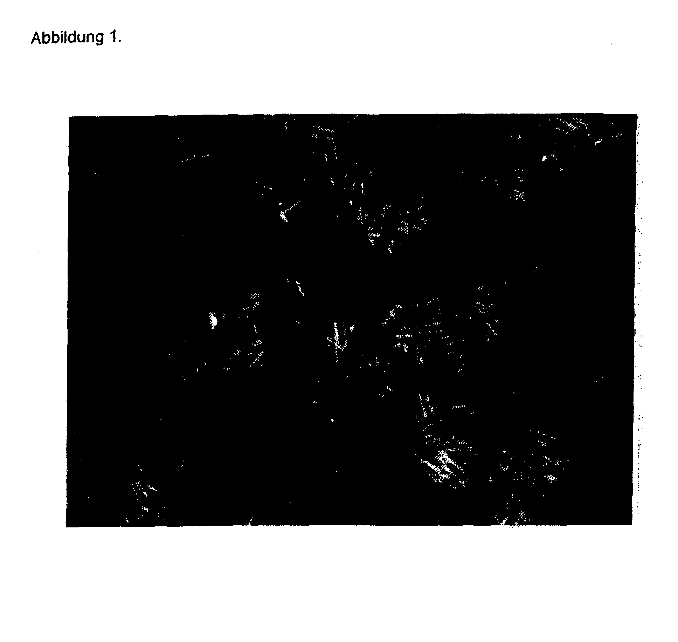

Für die Bindung an die Zellwand und den Nachweis von *Listeria* Zellen (z.B. *Listeria monocytogenes*, Stamm ScottA, Serovar 4b) wird eine dicht gewachsene Kultur (z.B. 1 ml) durch Zentrifigation konzentriert, in z.B. 500 µl des oben angegebenen Puffers resuspendiert, mit einer entsprechenden Menge (z.B. 20 µl, entsprechend z.B. 20 µg Protein) von GFP-CBD500 vermischt und 5 Minuten bei Raumtemperatur (z.B. 10-35° C) inkubiert. Anschließend werden die Bakterienzellen in einer Mikrozentrifuge zentrifugiert (z.B. 60 s bei 13.000 x g), der Überstand enthaltend das ungebundene GPP-CBD500 sorgfältig entfernt, und das Pellet in z.B. 500 µl des oben angegebenen Puffers resupendiert. Ein Aliquot (z.B. 10 µl) wird auf einen Objektträger gegeben, und mit Hilfe eines Epifluoreszenz-Mikroskopes unter Verwendung eines geeigneten Filtersatzes für die Abschirmung des UV-Lichtes betrachtet. Erfindungsgemäß zeigte sich, daß das Molekül sehr schnell und effizient an die Zellwand von Zellen der Gattung *Listeria* bindet; die gewünschten Eigenschaften der individuellen Moleküldomänen (fluoreszierende GFP Domäne, Zellwand-bindende CBD500 Domäne) bleiben somit vollständig erhalten. In Abbildung 1 ist die Bindung von GFP-CBD500 an Zellen von *Listeria monocytogenes* Stamm ScottA ersichtlich. Die Zellen wurden mit dem gereinigten Fusionsprotein vermischt, abzentrifugiert, mittels Zentrifugation gewaschen, und eine kleine Probe mit Hilfe eines Epifluoreszenz-Mikroskopes wie oben angegeben betrachtet. Die *Listeria* Zellen sind deutlich grün-leuchtend (in der schwarz-weiß Abbildung hell leuchtend) markiert. Der Bereich von Aminosäure His133 bis Lys289 bildet eine funktionsfähige CBD.

Mit ähnlichem Ergebnis läßt sich der Versuch unter Verwendung anderer CBD von *Listeria* Phagenlysinen wiederholen, z.B. unter Verwendung der CBD von Ply118 und CBD von Ply511. Allerdings lassen sich die CBD Domänen nicht beliebig voneinander ableiten, d.h., die funktionale CBD muß für jedes individuelle Protein aufgrund der speziellen Sequenz und sekundären/tertiären Proteinstruktur unabhängig voneinander neu bestimmt und getestet werden.

## Patentansprüche

1. Verfahren zur spezifischen Erkennung von Zielzellen durch Bindung, wobei das Verfahren folgende Schritte umfasst:
a) Auswahl von an die Zielzelle spezifisch bindenden Proteinen;
b) Bereitstellung der für die Zellwandbindung verantwortlichen Protein-Domänen (CBD) als Proteinfragmente, wobei diesen Proteinfragmenten jegliche hydrolytische Aktivität fehlt; und
c) Kontaktieren der in Schritt b) erhaltenen Protein-Domänen mit einer zu untersuchenden Probe

2. Verfahren gemäß Anspruch 1, wobei die an die Zielzelle spezifisch bindenden Proteine gewählt werden aus der folgenden Gruppe:
Bakteriophagen-kodierte Zellwandhydrolasen; bakterielle Zellwandhydrolasen; Autolysine; Rezeptormoleküle von Bakteriophagen und anderen für Hefen, Schimmel und eukaryontische Zellen spezifischen Viren; und mit der Zellwand nicht-kovalent assoziierte Zellwandproteine.

3. Verfahren gemäß Anspruch 2, **dadurch gekennzeichnet, dass** es sich um Endolysine, Phagenlysine, Lysine, Mureinhydrolasen, und/oder Peptidoglykanhydrolasen handelt.

4. Verfahren gemäß Anspruch 3, **dadurch gekennzeichnet, dass** die Lysine von Bakteriophagen für Bakterien der Gattung Listeria kodiert werden.

5. Verfahren gemäß einem oder mehreren der vorstehenden Ansprüche, **dadurch gekennzeichnet, dass** die Zielzellen gewählt werden aus der Gruppe, bestehend aus Bakterien und bakteriellen Sporen, Hefen, Schimmel und Schimmelsporen, pflanzlichen Zellen und tierischen Zellen.

6. Verfahren gemäß einem oder mehreren der vorstehenden Ansprüche, **dadurch gekennzeichnet, dass** die Zellwand-bindenden Polypeptid Domänen (CBD) aus der Nukleotidsequenz von Genen und/oder der Aminosäuresequenz von Genprodukten (Proteinen) abgeleitet und gewonnen werden.

7. Verfahren gemäß einem oder mehreren der vorstehenden Ansprüche, **dadurch gekennzeichnet, dass** die Genprodukte auch solche Genprodukte umfassen, welche erst nach posttranslationeller Modifikation funktionell und wirksam werden.

8. Verfahren gemäß einem oder mehreren der vorstehenden Ansprüche, **dadurch gekennzeichnet, dass** die verwendeten CBD durch genetische translationelle Fusionen direkt mit einem nachzuweisenden Marker verbunden sind.

9. Verfahren gemäß Anspruch 8, **dadurch gekennzeichnet, dass** es sich bei dem nachzuweisenden Marker um fluoreszierende Proteine, insbesondere GFP, BFP, besonders bevorzugt GFP mut-1, oder GFP mut-2 handelt.

10. Verfahren gemäß einem oder mehreren der vorstehenden Ansprüche, **dadurch gekennzeichnet, dass** die verwendeten CBD durch genetische, translationelle Fusionen direkt mit einem in weiteren Reaktionen nachzuweisenden Amplifikator verbunden sind.

11. Verfahren gemäß Anspruch 10, **dadurch gekennzeichnet, dass** der Amplifikator Peroxidase oder Phosphatase oder ein anderes Enzym mit vergleichbarer Wirkung ist.

12. Verfahren gemäß einem oder mehreren der vorstehenden Ansprüche, **dadurch gekennzeichnet, dass** die verwendeten CBD durch nachträgliche Modifikationen mit nachzuweisenden partikulären Markern, Farbstoffen, Amplifikatoren oder radioaktiven Isotopen versehen sind.

13. Verfahren gemäß Anspruch 12, wobei es sich bei dem Farbstoff um fluoreszente Farbstoffe handelt.

14. Verfahren gemäß Anspruch 12 oder 13, **dadurch gekennzeichnet, dass** es sich bei dem Amplifikator um Biotin, Peroxidase, Phosphatase oder vergleichbare Enzyme handelt.

15. Verfahren gemäß einem oder mehreren der vorstehenden Ansprüche, bei dem die CBD durch verschiedene chemische oder physikalische Verfahren und/oder Reaktionen an eine feste Phase gebunden werden, und nachfolgend durch Bindung an Zellwände der Zielzellen Immobilisierung der Zielzellen auf einer festen Oberfläche ermöglichen.

16. Verfahren gemäß Anspruch 15, wobei es sich bei der festen Phase um aktiviertes Polystyrol, aktiviertes Glas oder Silizium, aktiviertes Latex, Gold oder aktivierte Goldverbindungen, verschiedene ferromagnetische Trägermaterialien oder hydrophobe Kunststoffe handelt.

17. Verfahren gemäß einem oder mehreren der vorstehenden Ansprüche, **dadurch gekennzeichnet, dass** durch Festphasen-gebundenes CBD immobilisierte Zielzellen in der Art eines Sandwich-CBD Assays mit markierten und/oder modifizierten sekundären CBD Molekülen markiert und nachgewiesen werden können.

18. Verfahren gemäß einem oder mehreren der Ansprüche 1 - 16, **dadurch gekennzeichnet, dass** Festphasen-gebundenes CBD immobilisierte Zielzellen in der Art eines Sandwich-CBD-ELISA Assays mit markierten und oder modifizierten sekundären Antikörpern der Klasse der Immunglobuline markiert und nachgewiesen werden können.

19. Verfahren gemäß einem oder mehreren der Ansprüche 1 - 16, **dadurch gekennzeichnet, dass** durch Festphasen-gebundene primäre Antikörper der Klasse der Immunglobuline immobilisierte Zielzellen in der Art eines Sandwich-IG-CBD Assays mit markierten und/oder modifizierten sekundären CBD Molekülen markiert und nachgewiesen werden können.

20. Verfahren gemäß einem oder mehreren der Ansprüche 1 - 16, **dadurch gekennzeichnet, dass** an eine mobile Festphase gebundenes CBD Zielzellen aus verdünnten oder heterogenen Gemischen von Zellen heraus binden kann und in weiteren Schritten deren gezielte Anreicherung, Isolierung, Reinigung oder Nachweis ermöglicht.

21. Verfahren gemäß Anspruch 20, wobei es sich bei der mobilen Phase um ferromagnetische Partikel handelt.

22. Verwendung des Verfahrens gemäß einem oder mehreren der Ansprüche 1 - 21 zur Markierung, zum Nachweis, zur Diagnostik, zur Immobilisierung, und zur Anreicherung von Zellen.

23. Reagenzzusammenstellung für Verfahren gemäß einem oder mehreren der vorstehenden Ansprüche, enthaltend neben üblichen Nachweismitteln ein oder mehrere gemäß Schritt b) in Anspruch 1 definierte gereinigte CBD.

## Claims

1. Process for the specific recognition of target cells by binding, wherein the process comprises the following steps:
a) selection of proteins binding specifically to the target cell;
b) provision of the protein domains (CBD) responsible for cell-wall binding as protein fragments, wherein these protein fragments do not have any hydrolytic activity; and
c) contacting the protein domains obtained in step b) with a sample to be investigated

2. Process according to claim 1, wherein the proteins binding specifically to the target cell are selected from the following group:
bacteriophage-coded cell-wall hydrolases; bacterial cell-wall hydrolases; autolysins; receptor molecules of bacteriophages and other viruses specific for yeasts, moulds and eukaryotic cells; and cell-wall proteins associated non-covalently with the cell wall.

3. Process according to claim 2, **characterised in that** it is a question of endolysins, phagelysins, lysins, murein hydrolases, and/or peptidoglycan hydrolases.

4. Process according to claim 3, **characterised in that** the lysins are coded by bacteriophages for bacteria of the genus Listeria.

5. Process according to one or more of the above claims, **characterised in that** the target cells are selected from the group consisting of bacteria and bacterial spores, yeasts, moulds and mould spores, plant cells and animal cells.

6. Process according to one of more of the above claims, **characterised in that** the cell wall-binding polypeptide domains (CBD) are derived and recovered from the nucleotide sequence of genes and/or the amino acid sequence of gene products (proteins).

7. Process according to one or more of the above claims, **characterised in that** the gene products also include those gene products which only become functional and active after post-translational modification.

8. Process according to one or more of the above claims, **characterised in that** the CBD used are bound directly to a label to be detected by genetic translational fusions.

9. Process according to claim 8, **characterised in that** the label to be detected is fluorescing proteins, in particular GFP, BFP, particularly preferably GFP mut-1, or GFP mut-2.

10. Process according to one or more of the above claims, **characterised in that** the CBD used are bound directly to an amplifier to be detected in further reactions by genetic, translational fusions.

11. Process according to claim 10, **characterised in that** the amplifier is peroxidase or phosphatase or a different enzyme with comparable action.

12. Process according to one or more of the above claims, **characterised in that** the CBD used are provided with particulate labels to be detected, dyestuffs, amplifiers or radioactive isotopes, by subsequent modifications.

13. Process according to claim 12, wherein the dyestuff is fluorescent dyestuffs.

14. Process according to claim 12 or 13, **characterised in that** the amplifier is biotin, peroxidase, phosphatase or comparable enzymes.

15. Process according to one or more of the above claims, in which the CBD are bound to a solid phase by various chemical or physical processes and/or reactions, and enable immobilisation of the target cells on a solid surface subsequently by binding to cell walls of the target cells.

16. Process according to claim 15, wherein the solid phase is activated polystyrene, activated glass or silicon, activated latex, gold or activated gold compounds, various ferromagnetic support materials or hydrophobic plastics.

17. Process according to one or more of the above claims, **characterised in that** target cells immobilised by solid phase-bound CBD may be labelled and detected like a type of sandwich-CBD assay using labelled and/or modified secondary CBD molecules.

18. Process according to one or more of claims 1 - 16, **characterised in that** target cells immobilised by solid phase-bound CBD may be labelled and detected like a type of sandwich-CBD-ELISA assay using labelled and or modified secondary antibodies of the class of immunoglobulins.

19. Process according to one or more of claims 1 - 16, **characterised in that** target cells immobilised by solid phase-bound primary antibodies of the class of immunoglobulins may be labelled and detected like a type of sandwich-IG-CBD assay using labelled and/or modified secondary CBD molecules.

20. Process according to one or more of claims 1 - 16, **characterised in that** CBD bound to a mobile solid phase may immobilise target cells from dilute or heterogeneous mixtures of cells and in further steps enables their specific enrichment, isolation, purification or detection.

21. Process according to claim 20, wherein the mobile phase is ferromagnetic particles.

22. Use of the process according to one or more of claims 1 to 21 for labelling, for detection, for diagnostics, for immobilisation, and for enrichment of cells.

23. Reagent combination for processes according to one or more of the above claims, containing, in addition to conventional detection agents, one or more CBD purified as defined according to step b) in claim 1.

## Revendications

1. Un procédé d'identification spécifique de cellules cibles par liaison, le procédé comprenant les étapes suivantes :
a) sélection de protéines se liant de façon spécifique à la cellule cible ;
b) préparation des domaines de protéines (CBD) responsables de la liaison à des parois cellulaires, sous forme de fragments de protéines, ces fragments de protéines manquant de toute activité hydrolytique ; et
c) mise en contact des domaines de protéines obtenus à l'étape b) avec un échantillon à examiner.

2. Procédé selon la revendication 1, les protéines se liant spécifiquement à la cellule cible étant choisie dans le groupe suivant : hydrolases de parois cellulaires, à codage bactériophage ; hydrolases de parois cellulaires bactériennes ; autolysine, molécules réceptrices de bactériophages et autres virus spécifiques pour des levures, des moisissures et des cellules eucaryotes; et des protéines de paroi cellulaire associées de façon non covalente à la paroi cellulaire.

3. Procédé selon la revendication 2, **caractérisé en ce qu'**il s'agit d'endolysine, phagène-lysine, lysine, d'hydrolases muréiniques et/ou d'hydrolases peptidoglycanes.

4. Procédé selon la revendication 3, **caractérisé en ce que** la lysine est codée par des bactériophages pour des bactéries du genre Listéria.

5. Procédé selon l'une des revendications précédentes, **caractérisé en ce que** les cellules cibles sont choisies dans le groupe formé de bactéries et de spores bactériens, des levures, des moisissures et des spores de moisissures, des cellules végétales et des cellules animales.

6. Procédé selon une ou plusieurs des revendications précédentes, **caractérisé en ce que** les domaines de polypeptide (CBD) se liant à la paroi cellulaire sont dérivés et obtenus de à partir la séquence de nucléotides de gènes et/ou de la séquence d'acides aminés de produits génétiques (protéines).

7. Procédé selon une ou plusieurs des revendications précédentes, **caractérisé en ce que** les produits génétiques comprennent également des produits génétiques fonctionnels et efficaces après avoir subi une modification post-translationnelle.

8. Procédé selon une ou plusieurs des revendications précédentes, **caractérisé en ce que** les CBD utilisés sont liés directement à un marqueur assurant le marquage, par des fusions génétiques translationnelles.

9. Procédé selon la revendication 8, **caractérisé en ce qu'**il s'agit, concernant le marqueur devant assurer le marquage, de protéines fluorescentes, en particulier de GFP, BFP, de façon particulièrement préférée de GFP mut-1 ou GFP mut-2.

10. Procédé selon une ou plusieurs des revendications précédentes, **caractérisé en ce que** les CBD utilisés sont reliés directement par des fusions génétiques, translationnelles à un amplificateur, devant être décelé dans d'autres réactions.

11. Procédé selon la revendication 10, **caractérisé en ce que** l'amplificateur est une peroxydase ou une phosphatase ou une autre enzyme ayant un effet comparable.

12. Procédé selon une ou plusieurs des revendications précédentes, **caractérisé en ce que** les CBD utilisés sont munis de marqueurs, de colorants, d'amplificateurs ou d'autres produits radioactifs particuliers, assurant des fonctions de marquage, ceci par des modifications effectuées a posteriori.

13. Procédé selon la revendication 12, s'agissant, concernant le colorant, de colorants fluorescents.

14. Procédé selon la revendication 12 ou 13, **caractérisé en ce qu'**il s'agit, concernant l'amplificateur, de biotine, peroxydase, phosphatase ou d'enzymes comparables.

15. Procédé selon une ou plusieurs des revendications précédentes, pour lequel les CBD sont liés à une phase solide par différents procédés et/ou réactions chimiques ou physiques, et permettent ensuite, par une liaison aux parois cellulaires des cellules cibles, d'obtenir une immobilisation des cellules cibles sur une surface fixe.

16. Procédé selon la revendication 15, s'agissant, concernant la phase solide, de polystyrène activé, de verre ou de silicium activé, de latex activé, d'or ou de combinaison d'or activées, de différents matériaux supports ferromagnétiques ou de matières synthétiques hydrophobes.

17. Procédé selon une ou plusieurs des revendications précédentes, **caractérisé par** des cellules cibles, immobilisées par des CBD liées à des phases solides, et peuvent être marquées et décelées à la manière d'un essai CBD en sandwich avec des molécules CBD secondaires marquées et/ou modifiées.

18. Procédé selon une ou plusieurs des revendications 1 à 16, **caractérisé en ce que** des cellules cibles immobilisées par des CBD, liées à des phases solides, peuvent être marquées et être décelées à la manière d'un essai ELISA-CBD en sandwich, avec des anticorps secondaires, marqués et/ou modifiés, de la classe de l'immmuno-globuline.

19. Procédé selon une ou plusieurs des revendications 1 à 16, **caractérisé en ce que**, des cellules cibles au moyen d'anticorps primaires, de la classe de l'immuno-globuline immobilisées à des phases solides, peuvent être marquées et décelées à la manière d'un essai IG-CBD sandwich avec des molécules CBD secondaires marquées et/ou modifiées.

20. Procédé selon une ou plusieurs des revendications 1 à 16, **caractérisé en ce que**, CBD liées à une phase solide mobile, peuvent lier des cellules cibles d'un mélange dilué ou hétérogène de cellules, et, lors d'étapes supplémentaires, peuvent effectuer, à dessein, leur enrichissement, isolation, épuration ou détection.

21. Procédé selon la revendication 20, s'agissant, concernant la phase mobile, de particules ferromagnétiques.

22. Utilisation du procédé selon une ou plusieurs des revendications 1 à 21, pour le marquage, la détection, le diagnostic, l'immobilisation et l'enrichissement de cellules.

23. Composition de réactif pour des procédés selon une ou plusieurs des revendications précédentes, contenant, outre des agents de détection usuels, un plusieurs CBD épuré, défini à l'étape b) de la revendication 1.
